# EUROPEAN PATENT APPLICATION

(11) **EP 3 406 211 A1**
(43) Date of publication of application: **28.11.2018**
(21) Application number: 17172982.5
(22) Date of filing: 26.05.2017
(51) Int. Cl.: A61B 17/68, A61B 17/84

(54) **A BONE FIXATION PLUG**

(71) Applicant: Skulle Implants OY, 20520 Turku (FI)
(72) Inventor: Vallittu, Pekka, 21620 Kuusisto (FI)
(74) Representative: Suominen, Kaisa Liisa

(57) **Abstract**

The present invention relates to a bone fixation plug, comprising a cylindrical part comprising a lateral surface structure made of fibres in the form of a mesh and filled with non-rounded particles capable of promoting bone growth and of mechanically interlocking with the surrounding bone; a first end part arranged at a first end of the cylindrical part and a second end part arranged at a second end of the cylindrical part, made of fibre-reinforced composite material, wherein the first end part is adapted to cover the first end of the cylindrical part; and a cable tie attached to the first end part, passing through the cylindrical part essentially along its central axis, passing through the second end part, attached to the second end part and protruding from the second end part for a distance d.

## Description

### FIELD OF THE INVENTION

The present invention relates to a bone fixation plug, useful in neurosurgery and in orthopaedic surgery.

### BACKGROUND AND OBJECTS OF THE INVENTION

In neurosurgery, it is a common practice to open the skull by drilling standard sized holes to the skull bones and thereafter saw the bone to remove it and have an opening to the brain tissues. Removed bone flaps are arranged back to the opening after the operation and healing phase after the operation. Fixation of the bone flap is typically made with fixation plates and screws or by metallic clips, which resemble pop rivets, commercialised under the trade name CranioFix ®. Shortcomings of these metallic clips are that they do not provide a scaffold for bone to grow and fill the drilling holes. Furthermore, their upper surface is on the surface of the bone which may cause cosmetic problems to the patient. These clips have the additional disadvantage that they tend to slip away from the position they have been arranged to and may thus cause additional bleeding. A further disadvantage is that metallic clips do not allow for the use of all imaging methods after the surgery.

Furthermore, it would be advantageous to provide an attachment means for bones that is easy to use in other type of surgery, namely in orthopaedics. For example, such an attachment means could be used when attaching a fragmented bone back to place or when attaching a replacement bone implant to an existing, fractured or otherwise damaged bone.

There exists thus a need to provide an improved alternative for attachment means used in surgery, which should remove or at least significantly reduce the above-mentioned drawbacks.

### SUMMARY OF THE INVENTION

The above-mentioned problems can be at least partially and preferably fully overcome by the present invention. Indeed, the present description relates to a bone fixation plug, comprising
- a cylindrical part comprising a lateral surface structure made of fibres in the form of a mesh and filled with non-rounded particles capable of promoting bone growth and of mechanically interlocking with the surrounding bone,
- a first end part arranged at a first end of the cylindrical part and a second end part arranged at a second end of the cylindrical part, made of fibre-reinforced composite material, wherein the first end part is adapted to cover the first end of the cylindrical part and
- a cable-tie attached to the first end part, passing through the cylindrical part essentially along its central axis, passing through the second end part and protruding from the second end part for a distance d.

### BRIEF DESCRIPTION OF THE DRAWING

Figures 1A to 1C schematically illustrate use of a bone fixation plug according to a first embodiment.
Figure 2 schematically illustrates, as a lateral view, a skull with drill holes and sawing line to release the bone flap.
Figures 3A and 3B schematically illustrate use of a bone fixation plug according to a second embodiment.
Figures 4A and 4B schematically illustrate use of a bone fixation plug according to a third embodiment.

### DETAILED DESCRIPTION OF THE INVENTION

The present description relates to a bone fixation plug, comprising
- a cylindrical part comprising a lateral surface structure made of fibres in the form of a mesh and filled with non-rounded particles capable of promoting bone growth and of mechanically interlocking with the surrounding bone,
- a first end part arranged at a first end of the cylindrical part and a second end part arranged at a second end of the cylindrical part, made of fibre-reinforced composite material, wherein the first end part is adapted to cover the first end of the cylindrical part and
- a cable-tie attached to the first end part, passing through the cylindrical part essentially along its central axis, passing through the second end part and protruding from the second end part for a distance d.

The present bone fixation plug is thus based in the expansion of the cylindrical part of the plug when the plug is tightened. Indeed, the plug is placed for example in a hole left by drilling when the bone flap has been removed. The first end part is positioned underneath the bone and the second end part with the cable tie protruding from it on the upper side of the bone, i.e. towards the outside of the body. Then the cable tie is gently pulled and the cylindrical part thus deforms towards its exterior, i.e. expands. During this expansion, the particles inside the cylindrical part protrude partially from the mesh-like openings of the surface of the cylindrical part. Penetration of extracellular liquid, blood or saline lubricates the particles and allow them to move easier, to expand the cylindrical part and to protrude out of the cylindrical part. Protruded particles penetrate to the cancellous and compact bone of both the bone flap and the bone of the skull and thereby fix the bone flap in place, i.e. mechanically interlock the implant with the surrounding bone

The end parts of the plug hold the cylindrical part and are compressed to the surface of bone from each side. Thus, the fixation of the bone flap is based on increased resistance for shear forces by the protruded particles of the cylindrical part of the plug and compression by the end parts of the plug.

The present plug also takes advantage of the capillary effect, as the surface of the cylindrical part is formed of a mesh. Indeed, the structure is such that the capillary effect is enhanced, thus leading to improved bone ingrowth, as fluids can penetrate inside of the plug. The present bone fixation plug can also be used in various orthopaedic surgery in addition to neurosurgery, for example to fix an orthopaedic fracture fixation plate to a bone.

In this specification, by matrix, it is understood the continuous phase of a composition. By particles, it is meant entities wherein the largest dimension is no more than five times larger than the smallest dimension. The lateral surface of the cylindrical end part may also be called the mantle or the surface of the cylindrical part or the cylinder, and these are to be construed as having the same meaning. By a first end part is meant the part that is typically on the side of the brain in neurosurgery and on the side of the bone in orthopaedics. By the second end part is thus meant the part that is towards the outside of the skull or other bone.

According to an embodiment, the first end part and the second end part have an essentially circular cross-section. The end parts may also have other forms, such as rectangular or triangular. The largest diameter of the cross-section of the end part may be either essentially identical to the cross-section of the cylindrical part or smaller or larger than the cross-section of the cylindrical part. According to an embodiment, suitable especially for neurosurgery, the end parts both have a circular cross-section and their diameter is slightly larger than the diameter of the cylindrical part. The cylindrical part has a diameter that essentially corresponds to the diameter of the drilled hole. In case the second end part has a bevelled form, its end facing the cylindrical part has a diameter essentially matching the diameter of the cylindrical part and its other end has a diameter essentially matching the diameter of the drilled hole at the outer surface of the skull bone.

In especially neurosurgery, drilling of holes into the skull for enabling to saw the bone flap for its removal is typically made with standard sized burs, usually of 11 or 14 mm in diameter. The burs have a geometry which automatically forms a bevelled margin to the hole. Thus the second end part may have a bevelled form, i.e. it may be formed to adapt to the bevelled margin of the hole to ensure smooth, continuous surface from the bone flap to the plug and the surrounding skull bone and thus, not cause any cosmetic problems.

According to another embodiment, especially suitable for use in orthopaedic surgery where the bone fixation plug is integrally formed to the implant, the second end part has a diameter that is smaller than the diameter of the second end of the cylindrical part. In this embodiment, the second end part may be arranged inside the restorative implant in such a manner that only the cable tie protrudes out from the implant. An example of such an embodiment is provided below in the experimental part. The implant may be for example suitable for reconstructive or bone fragment fixation.

The cable-tie is made of a material that is strong enough to stand the pulling to bring the end parts closer to each other. It passes through the second end part and after fixation of the plug, it may be attached to the second part by a biocompatible glue or similar. According to another embodiment, the cable tie may be in the form of a cable tie, wherein the second end part is arranged to lock the cable tie into place (i.e. the cable tie itself is equipped with indentations and the second end part comprises corresponding indentations).

The distance d is chosen such that the end of the cable tie can be used by the surgeon to pull the plug into the desired thickness. For example, it can have a length between 5 and 20 cm. The cable tie is typically cut after the plug has been firmly put into place, such that no cable tie protrudes from the plug.

The end parts and the lateral surface structure of the cylindrical part may be homogenous in their structure and materials or they may consist of different materials and/or properties at different locations. It is for example possible to vary one or more of the following: the mesh size, the matrix material, the amount of matrix, the fibre material, the fibre diameter or the particle material. Preferably however, the lateral surface of the cylindrical part has a uniform structure.

According to an embodiment, at least one of the end parts comprises a lateral protrusion. By lateral it is meant that the protrusion is in the plane of the end part that is parallel to the end surface of the cylindrical part. The protrusion has a shape and size that makes it suitable for insertion into the incision left by sawing the bone flap. For example the protrusion can have a length of 2-5 mm and a width of 1-3 mm. The thickness of the protrusion is preferably essentially identical to the thickness of the end part. The lateral protrusion is thus preferably an integral part of the end part. Either one or both of the end parts may be equipped with such lateral protrusions. When the protrusion is used in only one of the end parts, it is preferably in the second end part, i.e. the outer end part. When a bone flap is positioned back to place, it may be advantageous to use, for the first drill hole to be closed, a plug comprising such a protrusion. This enables the first plug to hold the bone flap into place while the other holes are being filled with plugs.

According to an embodiment, the particles capable of promoting bone growth are selected from the group consisting of particles of bioactive glass, hydroxyapatite, tricalciumphosphate and mixtures thereof. Bioactive glasses suitable for such use are known in the literature and available on the market. One example of a suitable bioactive glass is known as the S53P4-glass and has a chemical composition of 53 weight-% of SiO₂, 23 weight-% of Na₂O, 20 weight-% of CaO and 4 weight-% of P₂O₅.

Depending on the stiffness of the fibre fabric used in the lateral surface of the cylindrical part, it may be made of the fabric alone or it may be a composite of the fibre fabric and a matrix polymer. According to an embodiment, the lateral surface structure of the cylindrical part further comprises a matrix. When a matrix polymer is used, the viscosity of the resin is such that it does not obstruct the mesh structure. Some examples of mesh size are given below.

The fibres of the lateral surface of the cylindrical part are preferably in the form of a fabric, either woven or non-woven. According to an embodiment, the fibres are used in the form of a woven fibre fabric which is positioned such that the fibres are arranged at an angle of 30-50 °, for example approximately 45 °, with respect to the longitudinal axis of the cylindrical part. The angle can be for example 25-70°, such as from 25, 30, 35, 40, 45, 50 or 55 ° up to 40, 45, 50, 55, 60, 65 or 70 °.

The fibres in the end parts may be in the form of fibre fabrics or fibre mats, and they may be oriented in two directions, three directions, four directions or randomly oriented.

The particles used in the cylindrical part are non-rounded, i.e. they have at least some sharp edges. The particles may also be in the form of chopped, short fibres.

The particles need to be such that they can partially protrude out from the cylindrical part when the plug is compressed. The size of the particles is larger than the mesh size of the lateral surface of the cylindrical part, in order for the cylindrical part to be able to retain them inside the plug. Some possible particles sizes are 10-1000 µm. The particle size can be for example from 10, 20, 50, 100, 150, 200, 250, 300, 400, 500, 650, 700 or 800 µm up to 20, 50, 100, 150, 200, 250, 300, 400, 500, 650, 700, 800, 900 or 1000 µm.

The amount of particles in the cylindrical part is such that the particles are able to move with respect to each other but also such that when the plug is compressed, i.e. the end parts are pulled closer to each other, the particles force the cylindrical part to expand laterally.

According to an embodiment, the mesh size of the lateral surface of the cylindrical part is optimized by weaving process of the mesh and viscosity and amount of impregnation resin of the mesh. According to an embodiment, the mesh size is preferably 1 to 5 micrometres less than the smallest diameter of the particles. The mesh size may be for example 9-999 µm. The mesh size may thus be for example from 1, 2, 3, 5, 7, 9, 10, 15, 20, 50, 100, 150, 200, 250, 300, 400, 500, 650, 700, 800 or 900 µm up to 2, 3, 5, 7, 9, 10, 15, 20, 50, 100, 150, 200, 250, 300, 400, 500, 650, 700, 800, 900, 950 or 1000 µm.

The plug may further comprise modifier particles. These modifier particles may for example be bioactive and for example improve the osteoconductivity of the plug. The particles may be in the form of particulate fillers or fibers. The weight fraction of these modifier particles in the plug can be for example 10-60 wt-%, such as from 5, 10, 15, 20, 35 or 50 wt-% up to 10, 15, 20, 35, 50, 55, 60 or 75 wt-%.

According to one embodiment, the modifier particles are selected from the group consisting of bioactive ceramics, bioactive glass, silica gel, titanium gel, silica xerogel, silica aerogel, natrium silica glass, titanium gels, bioactive glass ionomer, hydroxyapatite, Ca/P-doped silica gel and mixtures thereof. Any combination of said materials may naturally also be used. When rapid mineralization is needed, it is preferred to have bioactive glass with sol-gel processed silica particles.

The plug according to the present description may further comprise additional particulate filler material, such as metal oxides, ceramics, polymers and mixtures thereof. Metal oxides may for example be used as radio or X-ray opaque materials or as colouring materials.

The plug may also comprise therapeutically active agents or cells such as stem cells, proteins such as growth factors and/or signalling molecules. Several kinds of cells including hematopoietic bone marrow cells, fibroblasts, osteoblasts, regenerative cells, stem cells, like embryonic stem cells, mesenchymal stem cells or adipose stem cells can be seeded to the plug. The embryonic stem cells may or may not be of a human origin. Stem cells seeded to the plug can be cultured in bioreactors ex vivo, in other parts of the body before inserting the formed tissue into its final place, or directly at the place where regenerative and reconstructive treatment is needed. The plug may contain also additives enhancing its processability, such as polymerisation initiators. The materials of the plug can be either bioresorpable, biodegradable, biostable or a mixture of these.

All the materials used in the plug are naturally biocompatible and may be biodegradable or biostable.

The fibres used in the plug may be any suitable fibres known *per se,* for example selected from the group consisting of inert glass fibres, silica fibres, carbon/graphite fibres, inert ceramic fibres, aramid fibres, zylon fibres, polyethylene fibres, polytetrafluoroethylene fibres, such as Teflon® fibres, poly(p-phenylene-2,6-benzobisoxazole) fibres, poly(2,6-diimidazo(4,5-b4',5'-e)pyridinylene-1,4(2,5-dihydro)phenylene fibres, polyolefin fibres, fibres prepared from copolymers of olefins, polyester fibres, polyamide fibres and mixtures thereof. Poly(p-phenylene-2,6-benzobisoxazole) fibres and poly(2,6-diimidazo(4,5-b4',5'-e)pyridinylene-1,4(2,5-dihydro)phenylene fibres belong to a group called rigid-rod polymer fibres. It is obvious to a person skilled in the art that any other known fibres may be used in the present invention, provided that it is possible to obtain a suitable adhesion between said fibres and matrix, in order to achieve the desired mechanical properties and that the fibres are biocompatible.

According to one embodiment of the invention, the fibres are selected from the group consisting of inert glass fibres. According to another embodiment, the glass fibres are made of a glass composition of E-glass, S-glass, R-glass, C-glass or bioactive glasses. According to another embodiment, the fibres are selected from a group consisting of S-glass, E-glass, carbon fibres, aramid fibres and mixtures thereof.

According to yet another embodiment, the diameter of the fibres is 4-25 µm. The diameter of the fibres can be for example from 3, 5, 6, 10, 15, 20, 25, 30, 40, 45, 50, 60, 70 or 80 µm up to 5, 6, 10, 15, 20, 25, 30, 40, 45, 50, 60, 70, 80, 90 or 100 µm. Fibres in the nanometre scale, i.e. with a cross-sectional diameter varying between 200 - 1000 nm can also be used.

The matrix may be made of a biostable or bioresorbable resin consisting of monomers selected from the group consisting of methyl acrylate, ethyl acrylate, propyl acrylate, isopropyl acrylate, n-hexyl acrylate, styryl acrylate, allyl acrylate, methyl methacrylate, polymethyl methacrylate, ethyl methacrylate, propyl methacrylate, isopropyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diurethane dimethacrylate, acetoacetoxy ethyl methacrylate (AAEM), methacrylate functionalized dendrimers, other methacrylated hyperbranched oligomers, hydroxymethyl methacrylate, hydroxymethyl acrylate, hydroxyethyl methacrylate, hydroxyethyl acrylate, hydroxypropyl methacrylate, hydroxypropyl acrylate, tetrahydrofurfuryl methacrylate, tetrahydrofurfuryl acrylate, glycidyl methacrylate, glycidyl acrylate, triethylene glycol diacrylate, tetraethylene glycol dimethacrylate, tetraethylene glycol diacrylate, trimethylolethane trimethacrylate, trimethylolpropane trimethacrylate, pentaerythritol trimethacrylate, trimethylolethane triacrylate, trimethylolpropane triacrylate, pentaerythritol triacrylate, pentaerythritol tetramethacrylate, pentaerythritol tetra-acrylate, ethylene dimethacrylate, ethylene diacrylate, ethylene glycol dimethacrylate, diethylene glycol dimethacrylate, triethylene glycol dimethacrylate (TEGDMA), ethylene glycol diacrylate, diethyleneglycol diacrylate, butylene glycol dimethacrylate, butylene glycol diacrylate, neopentyl glycol dimethacrylate, hydroxyethyl methacrylate, urethan dimethacrylate, starburst methacrylated polyesters, hyperbranched methacrylated polyesters, neopentyl glycol diacrylate, 1,3-butanediol dimethacrylate, 1,3-butanediol diacrylate, 1,4-butanediol dimethacrylate, 1,4-butanediol diacrylate, 1,6-hexanediol dimethacrylate, 1,6-hexanediol diacrylate, di-2-methacryloxyethyl-hexametylene dicarbamate, di-2-methacryloxyethyl-trimethylhexametylene dicarbamate, di-2-methacryloxyethyl-dimethylbenzene dicarbamate, di-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene d icarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl-4-cyclohexyl carbamate, 2,2-bis(4-(2-hydroxy-3-methacryloxy)phenyl)propane (BisGMA), 2,2'-bis(4-methacryloxyphenyl)propane, 2,2'-bis(4-acryloxyphenyl)propane, 2,2'-bis[4(2-hydroxy-3-acryloxyphenyl)propane, 2,2'-bis(4-methacryloxyethoxyphenyl)propane, 2,2'-bis(4-acryloxyethoxyphenyl)-propane, 2,2'-bis(4-methacryloxypropoxyphenyl)propane, 2,2'-bis(4-acryloxy-propoxyphenyl)propane, 2,2'-bis(4-methacryloxydiethoxyphenyl)-propane, 2,2'-bis(4-acryloxydiethoxyphenyl)propane, 2,2'-bis[3(4-phenoxy)-2-hydroxypropane-1-methacrylate]propane, 2,2'-bis[3(4-phenoxy)-2-hydroxypropane-1-acrylate]propane, polyetheretherketone, polylactide, polycaprolactone, polyglycolide, their copolymers and mixtures thereof.

The matrix may naturally also consist of a mixture of a monomer(s) and a polymer(s).

According to one embodiment, the matrix material is an acrylate polymer. According to an embodiment, the matrix resin is selected from the group consisting of substituted and unsubstituted dimethacrylates and methacrylates. Some especially advantageous matrix materials (monomers) are methyl acrylate, methyl methacrylate, methacrylate functionalized dendrimers, glycidyl dimethacrylate (bisGMA), triethylene glycol dimethacrylate (TEGDMA) and urethane dimethacrylate (UDMA). The materials may be used as blends and they may form interpenetrating polymer networks (IPNs). They may also be functionalised with bioactive molecules that allow for a drug-like contact effect. Combinations of monomers and polymers are also suitable to be used, including modifications of resin systems by antimicrobial side group containing iodine which offers additional benefit in increasing radio opacity of the resin system.

According to an embodiment, the matrix is made of a resin selected from a group consisting of polyesters, epoxies, acrylates and mixtures thereof. According to another embodiment, the matrix resin is selected from the group consisting of substituted and unsubstituted dimethacrylates and methacrylates.

The plug according to this description can be manufactured in any suitable manner. One example of the manufacturing method is given below in the experimental part.

The present bone fixation plug can be used in connection with bone fracture plates or incorporated into bone fracture fixation plates. In this case, as is mentioned above, the second end part is embedded inside the fracture fixation plate and can thus have for example a smaller diameter than the diameter of the cylindrical part. In the case of a bone fracture plate, a drilling template is preferably provided, in order to ensure that fixation points are arranged at correct positions with respect to the fixation plate.

In surgery, the surgeon selects a plug which has a height of the cylindrical part that is approximately 2 mm more than thickness of the bone flap to be attached. The plug is preferably wetted with blood or a 0.9 % NaCl saline solution which lowers the friction between the particles inside the cylindrical part. When the plug and the bone flap are in place, the surgeon tightens the cable tie and thereby induces compression force to the discs toward surface of the bone flap and the margins of the bone. Simultaneously the cylindrical part expands due to lateral movement of the bioactive glass particles and the outermost particles protrude out from the mesh-like holes of the cylindrical part and penetrate the surface of the bone. The bone flap is thus fixed to the bone and the particles provide osteostimulating scaffold for bone to grow to the cylinder and form a biological bone bridge between the bone flap and the bone.

Some embodiments of the invention are explained in more detail in the enclosed drawing, which is not to be construed as limiting the claims. The reference signs are also not to be construed as limiting the claims.

### EXPERIMENTAL PART

### Example 1

Two glass fibre-reinforced composites discs having a thickness of 2 mm and a diameter of 16 mm were prepared of woven S-glass fabric (220 g/m2) and impregnated with bisGMA-TEGDMA resin matrix. After curing, one of the discs was finalized to adapt it to a bevelled hole of 14 mm in diameter. A medical grade cable tie was fixed to the other disc by using the resin system and curing. A cylindrical tube of mesh-like S-glass fabric with mesh holes of 0.4 mm in diameter and having a height that is 2 mm more than the thickness of the bone flap was prepared. The wall thickness of the cylindrical tube was is adjusted, with the thickness of the glass fabric and the resin, to be 0.1 mm. The fibres of the tube were aligned in +/- 45 degrees angle with respect to the axis of the cylindrical part. The cylindrical tube was attached, by the resin system and curing, to the non-bevelled disc such that the cable-tie passed through the cylindrical part. Thereafter, the cylindrical part was filled with particles of bioactive glass (S53P5) having a particle size between 0.5-0.8 mm and the bevelled disc was attached to both the cable-tie and the end of the cylindrical part.

### Example 2

A bone fracture fixation plate for tibia plateau fracture was made of glass fibre reinforced composite. Bevelled holes having a diameter of 6.0 mm were drilled to the plate and corresponding plugs with a cylindrical part (outer diameter 5.5 mm, length 20.0 mm) were prepared as in Example 1, except that the first end part had the same diameter than the cylindrical part (5.5 mm) and the second end part had bevelled margins having a diameter of 7.5 mm.

For drilling holes for the plugs to the fractured bone, a drilling template of same shape and indicating the location of holes which are in the actual plate was used. The fixation plate was positioned on the drilling holes, the plugs were wetted with blood and inserted to the holes and the cable ties are used to tighten, whereby the first end part moves closer to the second end part and the cylindrical part expands and attaches to the bone.

### Example 3

A bone fracture fixation plate for tibia plateau fracture was made of glass fibre reinforced composite. In the location of screw holes of a traditional fracture plate, cylindrical parts (having a diameter of 5.5 mm and a length 20.0 mm) were arranged, having the shape and structure as described above. The first end part having the same diameter as the cylindrical part was fixed to the cable tie which goes through the cylinder filled with bioactive glass and penetrates the fixation plate where the second end part of the plug was embedded. A drilling template for the surgeon to drill the holes to the corresponding locations in bone was provided. Once the surgeon is ready to position the fixation plate, it is placed such that the cylindrical parts and the first end part are arranged in the drill holes. The cable tie is then tightened and the fixation plate thereby attached to the tibia.

### Example 4

A cylindrical part of a plug was manufactured from biostable S-glass fibres and a bioresorbable polymer matrix which binds the fibres together. The thermoplastic polymer polylactide has a ductile polymer structure with high elongation percentage at break (6 %) and can be used as a polymer matrix of the cylindrical part. The polylactide was heated to the temperature of 160 °C and an S-glass fibre weave was impregnated with the molten polymer. The amount of polymer was kept at a minimum to impregnate the fibre rovings of the weave while leaving the mesh holes of the weave open. After impregnation of the weave, a cylinder with outer diameter of 14 mm was rolled from the polylactide impregnated fibre composite and cut to the length of 6 mm to be used in a bone flap fixation plug having a final length of 4 mm.

The cylindrical tube was attached, by the resin system and curing, to the non-bevelled biostable disc such that a cable-tie passed through the cylindrical part. Thereafter, the cylindrical part was filled with particles of bioactive glass (S53P5) having a particle size between 0.5-0.8 mm and the bevelled disc was attached to both the cable-tie and the end of the cylindrical part. When the plug was used, high elongation percentage of the polylactide enabled deformation of expansion of the cylindrical part to occur when the cable-tie was tightened. With time, the bioresorbable polylactide will resorb and the S-glass fibres remain in contact with the existing bone surface (at the time of the operation) and new bone formed after the operation.

### DETAILED DESCRIPTION OF THE DRAWING

In the following, the same reference signs are used of the same or similar components in different embodiments and/or Figures.

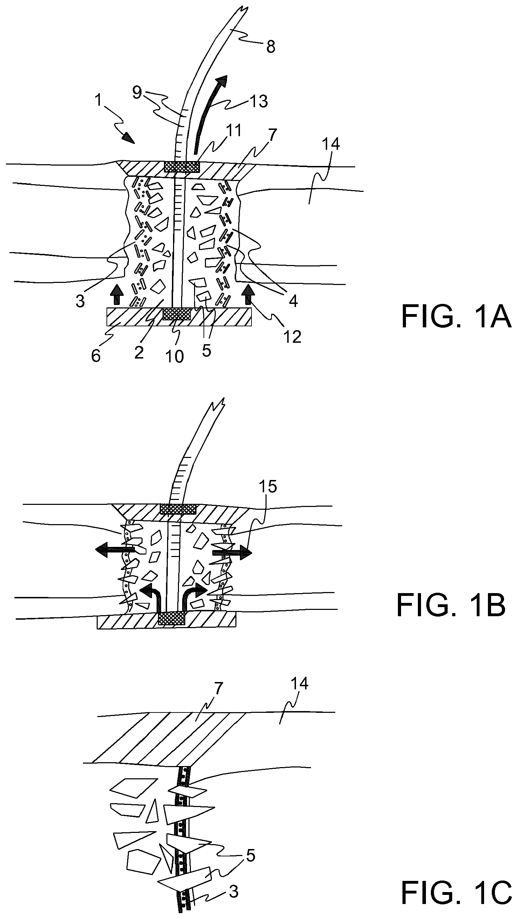

Figures 1A to 1C schematically illustrate use of a bone fixation plug according to a first embodiment. In Figure 1A, the plug 1 is in its initial position, i.e. as it has been arranged in the drill hole in the bone 14 to be filled. The Figure shows the cylindrical part 2 with its lateral surface structure 3 comprising openings 4 formed by the mesh. The cylindrical part 2 is filled with particles 5. Both ends of the cylindrical part 2 are closed by end parts, a first end part 6 and a second end part 7. A cable tie 8, which in this embodiment is in the form of a cable tie, comprises indentations 9. The cable tie 8 is attached to the first end part 6 at location shown with reference number 10 and passes through the second end part 7 at location shown with reference number 11.

In Figure 1A the plug is thus shown before it has been tightened into place. As can be seen, the plug is slightly longer than the thickness of the bone 1. The arrows 12, 13 show the direction of movement of the first end part 6 when the plug is tightened. The tightened plug is shown in Figure 1B, where the arrow 15 indicates the direction the cylindrical part expands, i.e. the lateral movement of the particles inside the cylindrical part. Figure 1C shows a closer look at the interface between the plug and the bone 1, where it can be seen that the particles 5 have protruded through the mesh structure of the lateral surface of the cylindrical part and penetrated into the bone.

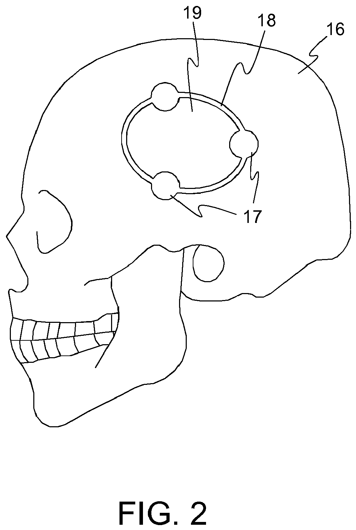

Figure 2 schematically illustrates, as a lateral view, a skull 16 with drill holes 17 and sawing line 18 to release the bone flap 19.

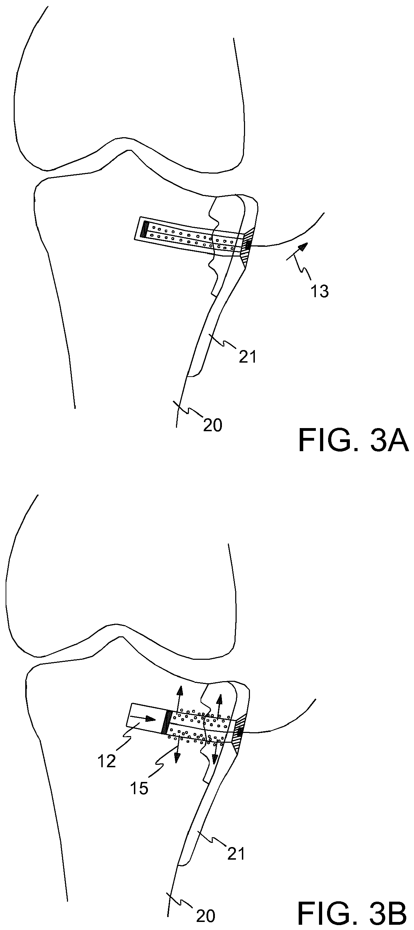

Figures 3A and 3B schematically illustrate use of a bone fixation plug according to a second embodiment. In these Figures, the plug is used to fix tibia plateau fracture plate 21 to a fractured tibia 20. Figure 3A shows the situation before tightening the cable tie and Figure 3B after tightening. The arrows 12, 13 and 15 illustrate the directions of pull for arrows 12 and 13 and the direction of expansion of the cylindrical part for arrow 15. The end parts of the plug are larger than the drill holes in the tibia.

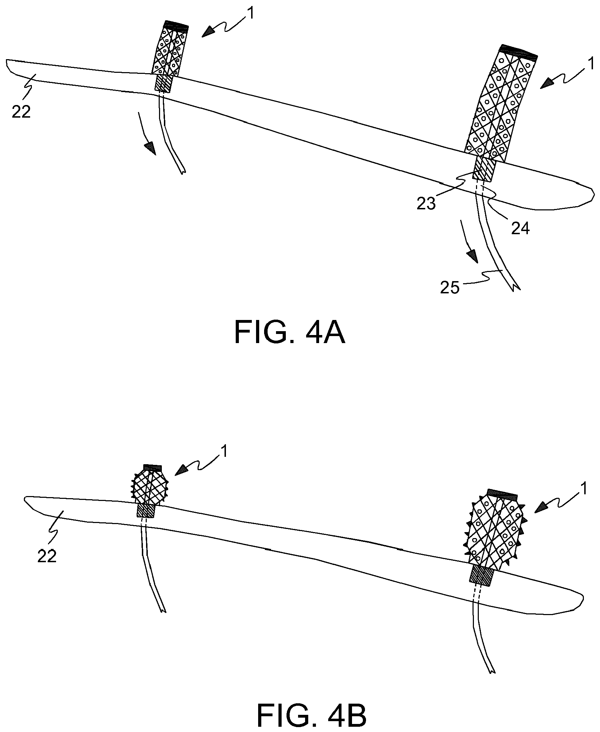

Figures 4A and 4B schematically illustrate use of a bone fixation plug according to a third embodiment. In this embodiment, the plug 1 is an integral part of the fracture plate 22. As can be seen, the second end plate 23 has a diameter that is smaller than the diameter of the cylindrical part and it is embedded in the fracture plate. The fracture plate is naturally also equipped with a small bore 24 through which the cable-tie 25 can pass. Figure 4A shows the situation before tightening the plug and Figure 4B the situation after tightening the plug.

## Claims

1. A bone fixation plug, comprising
- a cylindrical part comprising a lateral surface structure made of fibres in the form of a mesh and filled with non-rounded particles capable of promoting bone growth and of mechanically interlocking with the surrounding bone,
- a first end part arranged at a first end of the cylindrical part and a second end part arranged at a second end of the cylindrical part, made of fibre-reinforced composite material, wherein the first end part is adapted to cover the first end of the cylindrical part and
- a cable tie attached to the first end part, passing through the cylindrical part essentially along its central axis, passing through the second end part and protruding from the second end part for a distance d.

2. A bone fixation plug according to claim 1, wherein the cable tie is in the form of a cable tie and the second end part is arranged to lock the cable tie.

3. A bone fixation plug according to claim 1 or 2, wherein at least one of the end parts comprises a lateral protrusion.

4. A bone fixation plug according to any of the preceding claims, wherein the first end part and the second end part have an essentially circular cross-section.

5. A bone fixation plug according to claim 4, wherein the second end part has a bevelled form.

6. A bone fixation plug according to claim 4 or 5, wherein the second end part has a diameter that is smaller than the diameter of the second end of the cylindrical part.

7. A bone fixation plug according to any of the preceding claims, wherein the particles are selected from the group consisting of bioactive glass, hydroxyapatite, tricalciumphosphate and mixtures thereof.

8. A bone fixation plug according to any of the preceding claims, wherein the mesh size of the lateral structure of the cylindrical part is less than the size of the smallest particle inside the cylinder.

9. A bone fixation plug according to any of the preceding claims, wherein the fibres in the lateral surface structure of the cylindrical part are arranged at an angle of 30-50 ° with respect to the central axis of the cylindrical part.

10. A bone fixation plug according to any of the preceding claims, wherein the lateral surface structure of the cylindrical part further comprises a matrix.

11. A bone fixation plug according to any of the preceding claims, wherein the fibre-reinforced composite material comprises a matrix made of a resin selected from a group consisting of polyesters, epoxies, acrylates and mixtures thereof.

12. A bone fixation plug according to claim 11, wherein the matrix resin is selected from the group consisting of substituted and unsubstituted dimethacrylates and methacrylates

13. A bone fixation plug according to claim 11, wherein the matrix resin is selected from the group consisting of bioresorbable polymers of polylactides, polycaprolactones and polyglycolides.

14. A bone fixation plug according to any of the preceding claims, wherein the fibres of the cylindrical part and the end parts are independently selected from a group consisting of S-glass, E-glass, carbon fibres, aramid fibres and mixtures thereof.
